# EUROPEAN PATENT SPECIFICATION

(11) **EP 1 815 850 B1**
(45) Date of publication and mention of the grant of the patent: **26.11.2008**
(21) Application number: 06250124.2
(22) Date of filing: 11.01.2006
(51) Int. Cl.: A61K 9/28, A61K 9/16, A61P 25/06, A61K 31/19, A61K 9/48

(54) **Controlled release formulation of divalproic acid and its derivatives**
Formulierung zur verzögerten Freisetzung von Valproinsäure und deren Derivate
Formulation à libération contrôlée comprenant acide valproique et ses dérivés

(43) Date of publication of application: 08.08.2007
(60) Divisional application: 08007765.4
(73) Proprietor: TEVA PHARMACEUTICAL INDUSTRIES LTD., 49131 Petah Tiqva (IL)
(72) Inventor: Shterman, Nava, Petach Tikva, 49214 (IL); Ari-Pardo, Limor, (IL); Zilberman, Rina, Kfar Sava (IL); Triger-Messer, Yonit, Tel-mond (IL)
(74) Representative: Clyde-Watson, Zöe

(56) References cited:
- WO-A-01/39747
- US-A- 5 049 586
- US-B1- 6 287 598
- BRUCE L D ET AL: "PROPERTIES OF ENTERIC COATED SODIUM VALPROATE PELLETS" INTERNATIONAL JOURNAL OF PHARMACEUTICS, AMSTERDAM, NL, vol. 264, no. 1/2, 2 October 2003 (2003-10-02), pages 85-96, XP001180458 ISSN: 0378-5173
- QIU Y ET AL: "ONCE-A-DAY CONTROLLED-RELEASE DOSAGE FORM OF DIVALPROEX SODIUM I: FORMULATION DESIGN AND IN VITRO/IN VIVO INVESTIGATIONS" JOURNAL OF PHARMACEUTICAL SCIENCES, AMERICAN PHARMACEUTICAL ASSOCIATION. WASHINGTON, US, vol. 92, no. 6, June 2003 (2003-06), pages 1166-1173, XP001161543 ISSN: 0022-3549

## Description

### FIELD OF THE INVENTION

The present invention relates to pharmaceutical formulations. More particularly, the present invention concerns a formulation comprising valproic acid, a pharmaceutically acceptable salt, ester, or amide thereof or divalproex sodium, in a controlled release tablet formulation.

### BACKGROUND OF THE INVENTION

2-Propylpentanoic acid, more commonly known as valproic acid (VPA), its amide, valpromide (VPO), and certain salts and esters of the acid are effective in the treatment of epileptic seizures or as antipsychotic agents. Meade, U.S. Pat. No. 4,988,731, describes an oligomer having a 1:1 molar ratio of sodium valproate and valproic acid containing 4 units, and Meade, U.S. Pat. 5,212,326, describes a stable, non-hygroscopic solid form of valproic acid which comprises an oligomer having 1:1 molar ratio of sodium valproate and valproic acid and containing four to six units. Divalproex sodium (sodium hydrogen divalproate) is useful in the prophylaxis of migraine headaches in adults and may be used in the treatment or prophylaxis of seizures.

However, despite its efficacy in the treatment of epilepsy, valproic acid has been shown to exhibit an elimination half-life which is apparently shorter than other commonly used anti-epileptic agents. Half-lives for the drug of between six and seventeen hours in adults and between four and fourteen hours in children have been reported. This can lead to substantial fluctuations in the plasma concentration of the drug, especially in chronic administration. To maintain reasonably stable plasma concentrations, it is perhaps necessary to resort to frequent dosing, and the resulting inconvenience to the patient often results in lowered compliance with the prescribed dosing regimen. Moreover, widely fluctuating plasma concentrations of the drug may result in administration of less than therapeutic amounts of the drug in a conservative dosing regimen, or amounts too large for the particular patient in an aggressive dosing regimen.

To overcome this disadvantage valproic acid formulations which maintain more constant plasma levels of the drug following administration have been developed. The ultimate goal is the development of a formulation which affords stable plasma levels in an once-a-day dosing regimen. Efforts to accomplish this goal fall generally into one of two categories: (a) finding a form of the active ingredient which is more slowly released to the body metabolically, and (b) finding a formulation which delivers the drug by either a timed- or controlled-release mechanism.

U.S. Pat. No. 4,369,172 (Schor, et al.) describes, for example, a prolonged release therapeutic composition based on mixtures of hydroxypropyl methylcellulose, ethyl cellulose and/or sodium carboxymethyl cellulose. Schor et al provide a long list of therapeutic agents which they suggest can be incorporated into the formulation including sodium valproate.

U.S. Pat. No. 4,913,906 (Friedman, et al.) appears to describe a controlled release dosage form of valproic acid, its amide, or one of its salts or esters in combination with a natural or synthetic polymer, pressed into a tablet under high pressure.

U.S. Pat. No. 5,009,897 (Brinker, et al.) describes granules, suitable for pressing into tablets, the granules comprising a core of divalproex sodium and a coating of a mixture of a polymer and microcrystalline cellulose.

U.S. Pat. No. 5,019,398 (Daste) describes a sustained-release tablet of divalproex sodium in a matrix of hydroxypropyl methylcellulose and hydrated silica.

U.S. Pat. No. 5,055,306 (Barry, et al.) describes an effervescent or water-dispersible granular sustained release formulation suitable for use with a variety of therapeutic agents. The granules comprise a core comprising the active ingredient and at least one excipient, and a water insoluble, water-swellable coating comprising a copolymer of ethyl acrylate and methyl methacrylate and a water soluble hydroxylated cellulose derivative. The patentees suggest a list of therapeutic agents which may be used in the formulation of the invention, including sodium valproate.

U.S. Pat. No. 5,169,642 (Brinkler, et al.) appears to describe a sustained release dosage form comprising granules of divalproex sodium or amides or esters of valproic acid coated with a sustained release composition comprising ethyl cellulose or a methacrylic methyl ester, a plasticizer, a detackifying agent, and a slow-release polymeric viscosity agent.

U.S. Pat. No. 5,185,159 (Aubert, et al.) a formulation of valproic acid and sodium valproate which is prepared seemingly without the use of either a binder or a granulating solvent. The formulation optionally contains precipitated silica as an anti-sticking or detackifying agent.

U.S. Pat. No. 5,589,191 (Exigua, et al.) describes a slow release sodium valproate tablet formulation in which the tablets are coated with ethyl cellulose containing silicic acid anhydride.

Published PCT application WO 94/27587 (Ayer, et al.) describes a method for control of epilepsy by delivering a therapeutic composition of valproic acid or a derivative in combination with a poly(alkylene oxide).

Bialer, et al., "Metabolism of Antiepileptic Drugs," pp. 143-151, R. H. Levy, Ed., Raven Press, New York, 1984; Int. J. Pharmaceutics, 20: 53-63 (1984); and Biopharmaceutics and Drug Disposition, 6: 401-411 (1985); and Israel J. Med. Sci., 20: 46-49 (1995) report the pharmacokinetic evaluation of several sustained release formulations of valproic acid.

U.S. Pat No. 6,419,953 (Qiu et al.) appears to describe a hydrophilic matrix tablet suitable for the once-a-day administration of valproate compounds such as divalproex sodium, with hydroxypropylmethyl cellulose in an amount from about 20 weight percent to about 40 weight percent.

U.S. Pat. No. 6,528,090 (Qiu et al.) allegedly describes an oral controlled release formulation suitable for the once-a-day administration of valproate compounds, with a pharmaceutically acceptable hydrophilic polymer in an amount from about 20% to about 50% by weight of the formulation.

There remains, however, the need for a sustained release formulation of valproic acid which will effectively maintain plasma concentrations of the drug at more constant levels.

### SUMMARY OF THE INVENTION

The present invention provides a controlled release tablet dosage formulation comprising
a) about 40% to about 80% of a valproic acid compound selected from valproic acid, or a pharmaceutically acceptable salt, ester or amide thereof such as Divalproex Sodium, and
b) at least two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, each in an amount of from 10% to 16% of the tablet weight. Preferably the controlled release formulation comprises two to four polymers, more preferably 2 polymers. Preferably, the controlled release formulation comprises from about 45- about 55% of a valproic acid compound. Moreover, the polymers are preferably selected from the group consisting of hypromellose (Hydroxypropylmethyl cellulose HPMC), hydroxyethyl cellulose, and Polyethylene Oxide.

In another aspect the present invention also provides a controlled release tablet dosage form comprising a valproic acid compound and at least two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, each in an amount of from 10% to 16% by weight of the tablet, having a dissolution profile in an aqueous buffer at 37°C and pH 5.5 of
a) no more than about 30% of total valproate is released during or within 3 hours of measurement in said apparatus;
b) from about 35% to about 70% of total valproate is released during or within 9 hours of measurement in said apparatus;
c) from about 50% to about 95% of total valproate is released during or within 12 hour of measurement in said apparatus, and;
d) not less than 75% of total valproate is released during or within 18 hours of measurement in said apparatus.

Further, there is provided a method of preparing a granular composition suitable for pressing into controlled release tablets dosage form comprising the following steps of
a) dry blending a mixture of polymers comprising a valproic acid compound such as divalproex sodium, at least two, preferably two to four and more preferably two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, preferably hypromellose, and hydroxyethylcellulose, and a binder, preferably pregelatinized starch;
b) wet granulating with a hydro alcoholic solution, a mixture of an alcohol and water, in order to form a homogeneous mixture;
c) drying and sizing the wet granulate;
d) surface coating the dried granulate with a dispersion containing a hydrophilic polymer, preferably hypromellose, and talc, with purified water;
e) drying the coated granulate;
f) optionally adding a glidant, preferably silicon dioxide, and sieving the coated granulate;
g) optionally dry blending the coated granulate with a filler, preferably microcrystalline cellulose, and a lubricant preferably stearic acid;
h) compressing the blended granules into tablets; and
i) optionally coating the tablets with a cosmetic coat wherein each hydrophilic polymer in steps a) and d) is in an amount of 10% to 16% of the tablet weight.

### DETAILED DESCRIPTION OF THE INVENTION

As used herein, the terms "sustained release," "prolonged release," and "controlled release" as applied to drug formulations have the meanings ascribed to them in "Remington's Pharmaceutical Sciences," 18th Ed., p. 1677, Mack Pub. Co., Easton, Pa. (1990). Sustained release drug systems include any drug delivery system which achieves the slow release of drug over an extended period of time, and include both prolonged and controlled release systems. If such a sustained release system is effective in maintaining substantially constant drug levels in the blood or target tissue, it is considered a controlled release drug delivery system. If, however, a drug delivery system extends the duration of action of a drug over that achieved by conventional delivery, without reference to whether it is successful at achieving substantially constant blood or tissue drug levels, it is considered a prolonged release system.

The formulations of the present invention provide a controlled release formulation for valproic acid compounds. The term "valproic acid" is meant to encompass the compound 2-propylpentanoic acid per se, and its pharmaceutically acceptable salts, and compounds which readily metabolize in vivo to produce valproic acid, such as valproic acid amide (valpromide), as well as other pharmaceutically acceptable amides and esters of the acid. A particularly preferred form of valproic acid for the compositions of the present invention is the complex formed between 2-propylpentanoic acid and its sodium salt (2:1), commonly referred to as "divalproex sodium."

Divalproex Sodium may be represented by the following structure, wherein m is an integer from two to six:

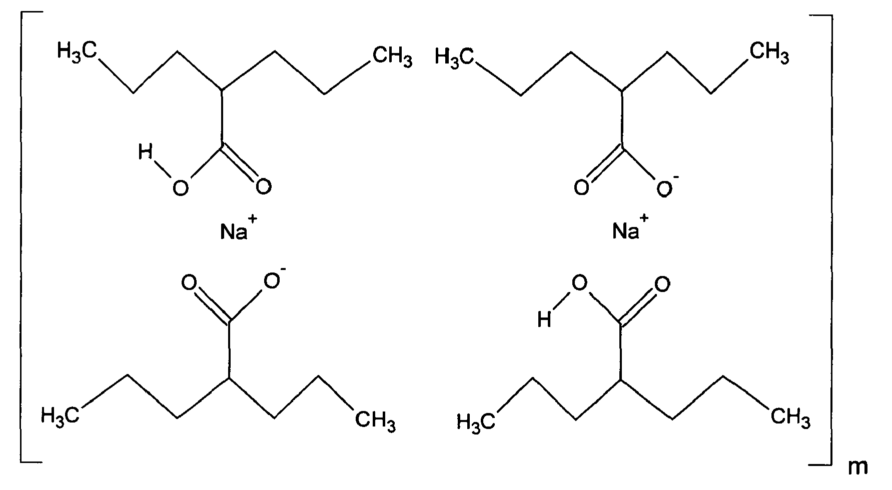

Methods for the synthesis of valproic acid are described in Oberreit, Ber. 29, 1998 (1896) and Keil, Z. Physiol. Chem. 282, 137 (1947). It's activity as an antiepileptic compound is described in the Physician Desk Reference, 52nd Edition, page 421 (1998). Upon oral ingestion within the gastrointestinal tract, the acid moiety disassociates to form a carboxylate moiety (i.e. a valproate ion). The sodium salt of valproic acid is also known in the art as an anti-epileptic agent. It is also known as sodium valproate and is described in detail in The Merck Index, 12th Edition, page 1691 (1996). Further descriptions may be found in the Physician Desk Reference, 52nd Edition, page 417 (1998).

Divalproex sodium is effective as an anti-epileptic agent and is also used for, migraine and bipolar disorders. Methods for its preparation may be found in U.S. Pat. Nos. 4,988,731 and 5,212,326. Like valproic acid, it apparently also disassociates within the gastrointestinal tract to form a valproate ion.

Furthermore, as used herein:
a) any reference to "valproic acid compounds" should be construed as including a compound which disassociates within the gastrointestinal tract, or within in-vitro dissolution media, to produce a valproate ion including, but not limited to, valproic acid, the sodium salt of valproate, divalproex sodium, any of the various salts of valproic acid described above, and any of the prodrugs of valproic acid described above. Divalproex sodium is the most preferred valproate compound of the present invention.
b) "Cₘₐₓ " means maximum plasma concentration of the valproate ion, produced by the ingestion of the composition of the invention.
c) "AUC" as used herein, means area under the plasma concentration-time curve, as calculated by the trapezoidal rule over the complete 24-hour interval for all the formulations.
d) "Pharmaceutically acceptable" as used herein, means those salts, polymers, and excipients which are, within the scope of sound medical judgment, suitable for use in contact with the tissues of humans and lower animals without undue toxicity, irritation, allergic response, and the like, in keeping with a reasonable benefit/risk ratio, and effective for their intended use in the treatment and prophylaxis of various disorders.

Further, in-vitro dissolution profiles are often used in the manufacture of pharmaceuticals. They serve as quality control devices to insure that different batches will have the same dissolution profile and thus produce comparable biological responses.

A new valproic acid compound dosage form is provided that possess significant advantages over the sustained release formulations of the prior art. These formulations provide substantially zero (0) order release of valproate, minimizing the variance between peak and trough plasma levels of valproate. All of the formulations of this invention comprise a matrix system comprising at least two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, each in an amount of from 10% to 16% of the tablet weight, and may further comprise a granulate matrix surface coating.

In a matrix system, the drug or active pharmaceutical ingredient is homogenously dispersed in a polymer in association with conventional excipients. This admixture may be compressed under pressure to produce a tablet. The drug is released from this tablet by diffusion and erosion. Matrix systems are described in detail by (1) Handbook of pharmaceutical controlled release technology, Ed. D. L. Wise, Marcel Dekker, Inc. New York, N.Y. (2000), and (2) Treatise on controlled drug delivery, fundamentals, optimization, applications, Ed. A. Kydonieus, Marcel Dekker, Inc. New York, N.Y. (1992).

The enhanced pharmacokinetic profile, described in more detail below, can be obtained by the administration of a matrix formulation of a valproic acid compound comprising at least two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers. In one embodiment, the controlled release dosage form according to the invention comprises:
a) a valproic acid compound, present in an amount sufficient to provide the required daily dose of valproate, and
b) at least two, preferably two to four and more preferably two, hydrophilic polymers, wherein each polymer is in an amount of from 10% to 16% of the tablet weight.

In another embodiment of the present invention there is provided a controlled release formulation comprising
a) a valproic acid compound, present in an amount sufficient to provide the required daily dose of valproate,
b) a pharmaceutical matrix granulate comprising at least two, preferably two to four and more two, preferably hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, and
c) a granulate surface coating of the pharmaceutical matrix granulate comprising a polymer, preferably a hydrophilic polymer, wherein the amount of each individual type of polymer in the formulation is from 1% to 16%, of the weight of the composition.

In preferred embodiments this surface coated granulate is pressed into a core which may be optionally coated with, preferably a cosmetic coat. In such an embodiment, the formulation can take the form of a coated core or a plurality of coated cores which may optionally be compressed into a tablet or administered, for example in a capsule.

Preferably the valproic acid compound is divalproex sodium. The amount of a valproic acid compound sufficient to provide the required daily dose of valproate varies from about 40% to about 80% by weight of the dosage form. More preferably, the controlled release dosage form of the present invention comprises about 45% to about 60% by weight of the valproic acid compound, most preferably about 45%-55% of the valproic acid compound.

Hydrophilic polymers for use in controlled release dosage forms are selected from the group consisting of hypromellose (HPMC, such as for example Methocel K100, Methocel E15, and Pharmacoat 606), hydroxyethyl cellulose (HEC, such as for example Natrosol® 250M), Polyethylene Oxide, polyvinylpyrrolidine, hydroxypropyl cellulose, methyl cellulose, vinyl acetate copolymers (such as for example Kollicoat SR 30, an aqueous dispersion of Polyvinyl acetate stabilized with polyvinylpyrrolidone and sodium lauryl sulfate), polysaccharides (such as alginate, xanthum gum, etc.), methacrylic acid copolymers, maleic anhydride/methyl vinyl ether copolymers and derivatives thereof. Preferably, the hydrophilic polymers are selected from hypromellose, hydroxyethyl cellulose, Polyethylene Oxide, and Kollicoat. Most preferably, the hydrophilic polymers are hypromellose and hydroxyethyl cellulose. Preferred hypromellose compounds include the commercially available Methocel K100, Methocel E15, and Pharmacoat 606. A preferred commercially available hydroxyethyl cellulose is Natrosol® 250M.

The amount of each polymer in the dosage formulation of the present invention is from 10% to 16% by weight of the dosage form. Most preferably, the amount of each polymer varies from about 12% to about 16% by weight of the dosage form.

The controlled release tablet dosage formulation of the present invention may further comprise a hydrophobic polymer such as for example ethylcellulose. Preferred commercially available ethylcelluloses include Ethocel Premium 7cps and Ethocel Premium 100cps.

The composition of the invention also typically includes pharmaceutically acceptable excipients. As is well known to those skilled in the art, pharmaceutical excipients are routinely incorporated into solid dosage forms. This is done to ease the manufacturing process as well as to improve the performance of the dosage form. Common excipients include diluents or bulking agents, lubricants, binders, etc. Such excipients are routinely used in the dosage forms of this invention.

Diluents, or fillers, are added in order to increase the mass of an individual dose to a size suitable for tablet compression. Suitable diluents include powdered sugar, calcium phosphate, calcium sulfate, microcrystalline cellulose, lactose, mannitol, kaolin, sodium chloride, dry starch, sorbitol, etc.

Lubricants are incorporated into a formulation for a variety of reasons. They reduce friction between the granulation and die wall during compression and ejection. This prevents the granulate from sticking to the tablet punches and dies, facilitates its ejection from the tablet punches, etc. Examples of suitable lubricants include talc, sodium lauryl sulfate, stearic acid, vegetable oil, calcium stearate, zinc stearate, magnesium stearate, sodium stearyl fumarate, etc.

Glidant's are also typically incorporated into the formulation. A glidant improves the flow characteristics of the granulation. Examples of suitable glidant's include talc, silicon dioxide, and cornstarch.

Binders may be incorporated into the formulation. Binders are typically utilized if the manufacture of the dosage form uses a granulation step. Examples of suitable binders include povidone, polyvinylpyrrolidone, xanthan gum, cellulose gums such as carboxymethylcellulose, methyl cellulose, hydroxypropylmethylcellulose, hydroxycellulose, gelatin, starch, and pregelatinized starch. Also, binders are often the same polymers as the polymers used to control the release of the active ingredient from the formulation.

Other excipients that may be incorporated into the formulation include preservatives, antioxidants, or any other excipient commonly used in the pharmaceutical industry, etc. The optional tablet coat is preferably cosmetic and may be prepared from, for example, commercially available powders for coating suspensions based on either Hypromellose or Polyvinyl alcohol, together with polyethylene Glycol and colorants etc.

In another embodiment of the present invention there is provided a controlled release tablet core dosage form comprising from about 47 to about 50 weight percent of a valproic acid compound; about 13 to 16 weight percent hypromellose; about 13 to 15 weight percent hydroxyethylcellulose; about 8 to 9 weight percent pregelatinized starch; about 4 to 10 weight percent microcrystalline cellulose; about 3 to 4 weight percent silicon dioxide and about 1 to 2 weight percent stearic acid; all weight percentages based upon the total weight of the tablet dosage form.

A particularly preferred controlled release formulation of the invention is described in Table 1 infra, wherein all weight percentages are based upon the total weight of the dosage form.

**Table 1: Preferred Controlled Release Formulation of the Invention (Uncoated Tablet Core)**

| Ingredient | Formulation (mg) | (%) of tablet |
|---|---|---|
| **CORE:** | | |
| Divalproex Sodium | 538.2 | 47.1-50.6 |
| Starch | 91.8 | 8.0-8.6 |
| Hydroxypropylmethyl Cellulose (Methocel K-100M) | 90.0-150.0 | 8.0-13.1 |
| Hydroxyethyl Cellulose NF | 150.0 | 13.1-14.1 |

| **SURFACE COATING:** | | |
|---|---|---|
| Hydroxypropylmethyl Cellulose (Methocel E-15) | 24.0-84.0 | 2.2-7.4 |
| Talc Extra Fine | 28.0 | 2.5-2.6 |

| **DRY MIXING:** | | |
|---|---|---|
| Silicon Dioxide (Syloid 244) | 40.0 | 3.5-3.8 |
| Microcrystalline Cellulose (Avicel PH 102) | 50.0 | 4.6-8.8 |
| Stearic Acid NF | 20.0 | 1.8-1.9 |
| Total | 1064.0-1142.0 | 100 |

In another embodiment of the present invention there is provided a controlled release tablet dosage formulation comprising at least two, preferably two to four and more preferably two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, each in an amount of from 10% to 16% of the tablet weight the formulation having a dissolution profile of a) no more than about 30% of the total valproic acid compound is released during or within 3 hours; b) from about 35% to about 70% of the total valproic acid compound is released during or within 9 hours; c) from about 55% to about 95% of the total valproic acid compound is released during or within 12 hour; and d) not less than 75% of the total valproic acid compound is released during or within 18 hours. The dissolution profile of the controlled release tablet dosage form of the present invention is in an USP type II apparatus at 100 rpm, at a temperature of 37°C, in an acid stage of 500 ml of 0.1N HCl for 45 minutes, followed by a basic stage of 900ml 0.05 M phosphate buffer with 75mM Sodium Lauryl Sulfate (SLS) pH 5.5.

In yet another embodiment of the present invention there is provided a controlled release tablet dosage formulation comprising a valproic acid compound and at least two, preferably two to four and more preferably two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, each in an amount of from 10% to 16% of the tablet weight, the formulation having a relative pharmacokinetic profile in a mammal as compared to the pharmacokinetic profile of Depakote ER (a commercially available extended release Divalproex Sodium formulation), of a) an average AUC value in the range from about 85% to about 120% wherein the observed AUC value for the Depakote ER is set at 100%, and b) a Cₘₐₓ value in the range from about 90% to about 160% wherein the observed Cₘₐₓ value for the Depakote ER is set at 100%, wherein the controlled release tablet dosage formulation is administered to a non-fasting mammal. Moreover, in a fasting mammal the AUC value ranges from about 90 to about 130 and the Cmax value ranges from about 100 to about 160. In this pharmacokinetic profile the parameters for AUC and Cmax are presented as 100 times the ratio of the observed value for the dosage formulation of the present invention versus the observed value for Depakote ER. Preferably, the values for the pharmacokinetic parameters of the dosage forms of the present invention satisfy the 0.80-1.25 criterion for equivalence with Depakote ER. The dosage formulations of the present invention comprising a valproic acid compound and at least two, preferably hydrophilic, polymers in an amount of less than 20%, preferably less than 16%, therefore have comparable pharmacokinetics in comparison to the commercially available Depakote ER formulation, and are preferably bioequivalent.

The controlled release formulations are generally prepared using standard techniques well known in the art. Typically, they are prepared by dry blending the, preferebly hydrophilic, polymer, filler, valproic acid compound, and other excipients followed by granulating the mixture using a hydro alcoholic solution (a mixture of alcohol and water) until proper granulation is obtained. The granulation is done by methods known in the art. The wet granules are dried in a fluid bed dryer, sifted and ground to appropriate size, and may subsequently be surface coated with a dispersion containing a hydrophilic polymer. Fillers and lubricating agents are mixed with the dried granulation to obtain the final formulation.

More specifically, a controlled release tablet dosage formulation comprising a valproic acid compound and at least two hydrophilic polymers may be prepared as in the following example. The method for preparing a granular composition suitable for pressing into controlled release tablets dosage form comprises the steps of,
1) dry blending a mixture of polymers comprising a valproic acid compound such as divalproex sodium, and a least two, preferably two to four and more preferably two hydrophilic polymers selected from the group consisting of hypromellose (HPMC), hydroxyethylcellulose (HEC), polyethylene oxide, polyvinylpyrrolidine, hydroxypropyl cellulose, methyl cellulose, vinyl acetate copolymers, polysaccharide methacrylic acid copolymers and maleic anhydride methyl vinyl ether copolymers, preferably hypromellose, and hydroxyethylcellulose, and binder, preferably pregelatinized starch;
2) wet granulating with a hydro alcoholic solution, a mixture of alcohol and water, in order to form a homogeneous mixture;
3) drying and sizing the wet granulate;
4) surface coating the dried granulate with a dispersion containing diluents and a hydrophilic polymer, more preferably hypromellose, and talc, with purified water;
5) drying the coated granulate;
6) adding a glidant, preferably silicon dioxide, and sieving the coated granulate;
7) dry blending the coated granulate with a filler, preferably microcrystalline cellulose, and a lubricant preferably stearic acid;
8) compressing the blended granules into tablets; and
9) optionally coating the tablets with a cosmetic coat wherein each hydrophilic polymer in step is 1) and 4) is in an amount from 10% to 16% of the tablet weight.

The compositions of the invention can be administered orally in the form of tablets, pills, or the granulate may be loose filled into capsules. The tablets can be prepared by techniques known in the art and contain a therapeutically useful amount of the valproic acid compound and such excipients as are necessary to form the tablet by such techniques. Tablets and pills can additionally be prepared with enteric coatings and other release-controlling coatings for the purpose of acid protection, easing swallow ability, etc. The coating may be colored with a pharmaceutically accepted dye. The amount of dye and other excipients in the coating liquid may vary and will not impact the performance of the extended release tablets. The coating liquid generally comprises film forming polymers such as hydroxypropyl cellulose, polyvinyl lalcohol, hydroxypropylmethyl cellulose, cellulose esters or ethers such as cellulose acetate or ethylcellulose, an acrylic polymer or a mixture of polymers. The coating solution is generally an aqueous solution or an organic solvent solution further comprising plasiticizer e.g. polyethylene glycol, propylene glycol, sorbitan monoleate, sorbic acid, colorants such as titanium dioxide, pharmaceutically acceptable dyes or lakes.

The formulations of the invention provide substantially level plasma concentrations of valproic acid falling within the therapeutic range of the drug over a period which permits administration once daily.

The following examples are presented in order to further illustrate the invention. These examples should not be construed in any manner to limit the invention.

### EXAMPLES

### Example 1: Divalproex Sodium formulations.

Tablets containing a tablet core of 538 mg of divalproex sodium, starch, and various polymers were prepared. The tablet compositions are presented in Table 2.

**Table 2: Divalproex Sodium controlled release formulations (mg/tablet)**

| **Materials** | **K-30965** | **K-32752** | **K-33429** | **K-33861** | **K-34999** | **K-35000** | **K-35002** | **K-35003** | **K-35689** | **K-36139** | **K-36143** |
|---|---|---|---|---|---|---|---|---|---|---|---|
| **Core** | | | | | | | | | | | |
| Divalproex Sodium | 538.2 | 538.2 | 538.2 | 538.2 | 538.2 | 538.2 | 538.2 | 538.2 | 538.2 | 538.2 | 538.2 |
| Starch 1500 | 55.0 | 90.0 | 149.8 | 89.8 | 91.8 | 91.8 | 91.8 | 91.8 | 91.8 | 91.8 | 91.8 |
| Polyox WSR N-12K | 90.0 | | 150.0 | | | | | | | | |
| Polyox WSR 301 | 110.0 | | | | | | | | | | |
| Hypromellose (Methocel K-15M CR) | | 150.0 | 150.0 | 150.0 | | | | | | | |
| Hypromellose (Methocel K-100M CR) | | | | | 90.0 | 90.0 | 150.0 | 150.0 | 90.0 | 90.0 | 150.0 |
| Ethocel Premium 7cps | 30.8 | | | 100.0 | | | | | | | |
| Hydroxyethylcellulose (Natrosol® 250M) | | 150.0 | | 150.0 | 150.0 | 150.0 | 150.0 | 150.0 | 150.0 | 150.0 | 150.0 |
| Calcium Phosphate Dibasic | 40.0 | | | | | | | | | | |

| **Surface coating** | | | | | | | | | | | |
|---|---|---|---|---|---|---|---|---|---|---|---|
| Hypromellose 6cps (Pharmacoat 606) | | | | | | 56.0 | | | | | |
| Hypromellose (Methocel E-15 CR) | | | | | 56.0 | | | | 56.0 | 84.0 | 24.0 |
| Ethylcellulose (Ethocel Premium 7cps) | | | | | | | 56.0 | | | | |
| Ethylcellulose (Ethocel Premium 100cps) | | | | | | | | 56.0 | | | |
| Dibutyl Sebacate | | | | | | | 7.0 | 7.0 | | | |
| PEG 400 | | | | | | | 7.0 | 7.0 | | | |
| Kollicoat SR 30D | | 46.0 | 50.0 | 40.0 | | | | | | | |
| TEC | | 17.3 | 18.75 | 15.0 | | | | | | | |
| Talc | | 15.6 | 16.25 | 13.0 | 28.0 | 28.0 | | | 28.0 | 28.0 | 28.0 |

| **Dry Mixing** | | | | | | | | | | | |
|---|---|---|---|---|---|---|---|---|---|---|---|
| Syloid | 60.0 | 40.0 | 40.0 | 40.0 | 40.0 | 40.0 | 40.0 | 40.0 | 40.0 | 40.0 | 40.0 |
| Avicel PH-102 | 70.0 | 50.0 | 50.0 | 50.0 | 50.0 | 50.0 | 50.0 | 50.0 | 50.0 | 100.0 | 50.0 |
| Magnesium Stearate | 10.0 | | | | | | | | | | |
| Stearic acid | | 20.0 | 20.0 | 20.0 | 20.0 | 20.0 | 20.0 | 20.0 | 20.0 | 20.0 | 20.0 |

| **Coating of Tablet** | | | | | | | | | | | |
|---|---|---|---|---|---|---|---|---|---|---|---|
| Talc | | | 7.8 | | | | | | | | |
| Pharmacoat 606 | | | 7.8 | | | | | | | | |
| Kollicoat SR 30D | | | 31.2 | | | | | | | | |
| PEG 6000 | | | 3.2 | | | | | | | | |
| Ethylcellulose (Ethocel Prem. 7cps) | | | | 3.0 | | | | | | | |
| TEC | | | | 13.5 | | | | | | | |
| Talc | | | | 3.0 | | | | | | | |
| Opadry 31F32870 Yellow | 25.0 | 35.0 | - | 10.5 | | | | | | | |
| Opadry II 85F27675 Grey | | | | 29.0 | | | | | 30.0 | | |
| **Total** | 1029.0 | 1152.1 | 1233.0 | 1265.0 | 1064.0 | 1064.0 | 1110.0 | 1110.0 | 1094.0 | 1142.0 | 1092.0 |
| % Hydroxyethyl cellulose(HEC) vs. tablet weight | | 13.02 | | 11.86 | 14.10 | 14.10 | 13.51 | 13.51 | 13.71 | 13.1 | 13.7 |
| % Hypromellose (HPMC) vs. tablet weight | | 13.02 | 12.16 | 11.86 | 13.72 | 13.72 | 13.51 | 13.51 | 13.35 | 15.2 | 15.9 |
| % Polyox vs. tablet weight | 19.40 | | 12.16 | | | | | | | | |
| % Kollicoat SR 30D | | 4.00 | 6.58 | 3.16 | | | | | | | |
| % Ethylcellulose | 3.00 | | | 8.14 | | | 5.06 | 5.06 | | | |

### Example 2: Preparation of Divalproex Sodium controlled release formulation.

This Example illustrate the manufacture of a preferred dosage form of the present invention on a scale of 2500 tablets.

Divalproex sodium was milled through in Quadro comill equipped with an 0.187 inch aperture screen. 2.691 kg of milled drug was loaded directly into a mixer and mixed with 230 g of starch NF, 225 g of Methocel K-100M (Hypromellose NF) and 375 g Natrosol® 250M (Hydroxyethylcellulose NF) for 4 minutes. This mixture was granulated using 150 g of 95% Alcohol USP for 1 minute and a mixture of 25 g purified water and 25 g 95% Alcohol for another 30 seconds, and subsequently dried. The dried granules were milled through a 1 mm aperture screen. The granules were then granulate coated in a fluidized bed drierusing a suspension prepared from 1350 g purified water and 280g Methocel E-15 (Hypromellose 15 cps NF) and 140 g talc. The coated granules were blended with 100 g of silicon dioxide (Syloid 244) and 125 g of microcrystalline cellulose (Avicel PH 102) in a mixer for 10 minutes. 50 g of Stearic Acid was added and mixed for another 5 minutes The blended mixture was compressed into 1.00 gram tablet cores.

### Example 3: Dissolution tests with Dilvaproex Sodium controlled release tablets.

Dissolution tests with the controlled release divalproex sodium tablet formulations were performed. These in vitro dissolution tests were conducted using an Apparatus II described in the United State Pharmacopeia XXI/National Formulary XVI. A comparative dissolution test in release medium was conducted under the following conditions. Divalproex Sodium ER tablets were dissolved in an USP type II apparatus at 100 rpm, at a temperature of 37°C, in an acid stage of 500 ml of 0.1N HCl for 45 minutes, followed by a basic stage of 900ml 0.05 M phosphate buffer with 75mM Sodium Lauryl Sulfate (SLS) pH 5.5. Sampling times in the basic stage were at 3, 9, 12, and 18 hours. The results of this dissolution test are presented in Table 3.

**Table 3: Percentage of labeled dose dissolved in a comparative dissolution test at the sampling times indicated in hours.**

| **Time, hours** | **K-30965** | **K-32752** | **K-33429** | **K-33861** | **K-34999** | **K-35002** | **K-35989** | **K-36076** |
|---|---|---|---|---|---|---|---|---|
| **0** | 0 | 0 | 0 | 0 | 0 | 0 | 0 | 0 |
| **0.75** | 4 | 2 | 1 | 2 | 3 | 2 | 2 | 3 |
| **3.75** | 20 | 13 | 25 | 23 | 22 | 17 | 29 | 19 |
| **9.75** | 59 | 39 | 60 | 52 | 58 | 42 | 59 | 47 |
| **12.75** | 98 | 51 | 80 | 64 | 68 | 58 | 67 | 55 |
| **18.75** | 102 | 87 | 95 | 81 | 85 | 84 | 81 | 71 |
| **24.75** | | | | | 96 | 102 | | |

Based upon these dissolution studies, the results appearing in Table 3 above, the following conclusions were drawn: a) no more than about 30% of total valproate is released during or within 3 hours of measurement in said apparatus; b) from about 35% to about 70% of total valproate is released during or within 9 hours of measurement in said apparatus; c) from about 55% to about 95% of total valproate is released during or within 12 hour of measurement in said apparatus, and; d) not less than 75% of total valproate is released during or within 18 hours of measurement in said apparatus.

### Example 4: In vivo pharmacokinetic study

In three in-vivo biostudies pharmacokinetics were compared between preferred formulations and a reference drug. The bioavailability and plasma concentration versus time profile of valproate from oral controlled release tablet formulations of divalproex sodium (K-34999, K-35002, and K-35689 described in Table 2) determined under fasting and non-fasting conditions were compared to those of a commercially available extended release divalproex sodium tablet formulation (Depakote ER) under the same conditions in healthy subjects (15 subjects in a non-fasting study and 13 in a fasting study).

Pharmacokinetic parameters were obtained for Cₘₐₓ and AUC₀₋₂₄ in both the fasted and non-fasted (fed) biostudy. Analyses of variance (ANOVAs) were obtained for these parameters as well. The pharmacokinetic results for each formulation as compared to the reference formulation are summarized in the Table 5.

**Table 5: Comparative Pharmacokinetics of formulation versus reference formulation**

| Formulation | Parameter | 100 XRatio of Geometric means | 90% CI on Log Transformed Data | Power of ANOVA for Log Transformed Data | P Value |
|---|---|---|---|---|---|
| K-34999 (fasted) | AUC | 105 | 93.4 - 118 | 0.79234 | 0.4793 |
| | Cₘₐₓ | 113 | 101 - 126 | 0.84891 | 0.0733 |
| K-35002 (fasted) | AUC | 115 | 102 - 130 | 0.79234 | 0.0500 |
| | Cₘₐₓ | 140 | 126 - 157 | 0.84891 | 0.0001 |
| K-35689 (fasted) | AUC | 107 | 98 - 117 | | |
| | Cₘₐₓ | 121.6 | 113.7 - 129.9 | | |
| K-34999 (fed) | AUC | 94 | 88.1-100 | 0.99828 | 0.1128 |
| | Cₘₐₓ | 103 | 94.1 - 112 | 0.96475 | 0.6119 |
| K-35002 (fed) | AUC | 106 | 98.9 - 113 | 0.99754 | 0.1670 |
| | Cₘₐₓ | 141 | 129 - 154 | 0.95639 | 0.0001 |
| K-35689 (fed) | AUC | 108 | 98.3 - 117.9 | | |
| | Cₘₐₓ | 117.5 | 110.6 - 124.8 | | |

The 90% confidence intervals for the AUC values for the test formulations administered under fasting and non-fasting conditions versus the reference fasting and non-fasting, would satisfy the criterion for bioequivalence when the values are in the range from 0.80-1.25 times the reference value. Additionally, the 90% confidence interval for the ratio of central values of Cₘₐₓ for the test formulations under fasting and non-fasting regimens would also be satisfied by the same bioequivalence criterion.

The controlled release tablet dosage formulations perform well. The controlled release regimens are either similar or bioequivalent to the reference regimen with respect to extent of absorption as characterized by AUC. The two test regimens did not differ statistically significantly from the reference regimen with respect to Cₘₐₓ values of the controlled release regimens compared the reference regimen suggest that the controlled release tablet dosage formulation provides controlled release of valproic acid in vivo under fasting and non-fasting conditions.

Moreover, the results demonstrate the controlled release characteristics of the test formulations and their similarity in bioavailability based on AUC when compared to the reference formulation.

## Claims

1. A controlled release tablet formulation comprising,
a) a valproic acid compound in an amount of about 40% to about 80% by weight of the dosage form, and
b) at least two hydrophilic polymers each in an amount of from 10% to 16% of the tablet weight,
wherein the valproic acid compound is selected from valproic acid, or a pharmaceutically acceptable salt, ester, or amide thereof, and the hydrophilic polymers are selected from the group consisting of hypromellose (HPMC), hydroxyethyl cellulose (HEC), polyethylene oxide, polyvinylpyrrolidine, hydroxypropyl cellulose, methyl cellulose, vinyl acetate copolymers, polysaccharides, methacrylic acid copolymers and maleic anhydride/methyl vinyl ether copolymers.

2. The formulation according to claim 1, wherein the valproic acid compound is in an amount of about 45% to about 60%.

3. The formulation according to claim 2, wherein the valproic acid compound is in an amount of about 45% to about 55%.

4. The formulation according to any preceding claim, wherein the amount of each hydrophilic polymer is from about 12% to about 16% of the tablet weight.

5. The formulation according to any preceding claim, wherein the hydrophilic polymers are selected from the group consisting of hypromellose (hydroxypropylmethyl cellulose, HPMC), hydroxyethyl cellulose and polyethylene oxide.

6. The formulation according to any preceding claim, wherein the hydrophilic polymers are selected from the group consisting of hypromellose (HPMC) and hydroxyethylcellulose.

7. The formulation according to any preceding claim, wherein the hydrophilic polymers are selected from the group consisting of hypromellose K100 (Methocel K100), hypromellose E15 (Methocel E15), and hydroxyethylcellulose 250 (Natrosol® 250M).

8. The formulation according to any preceding claim, further comprising at least one additional polymer which is hydrophobic.

9. The formulation according to claim 8, wherein the at least one hydrophobic polymer is ethylcellulose.

10. The formulation according to any preceding claim, wherein the formulation is in the form of a coated core, comprising
a) a compressed surface coated matrix granulate, and
b) a coating on the compressed core.

11. The formulation according to claim 10, wherein the surface coated matrix granulate comprises the valproic acid compound and at least two hydrophilic polymers.

12. The formulation according to any of claims 10-11, wherein the surface coating of the surface coated matrix granulate comprises a hydrophilic polymer

13. The formulation according to claim 12, wherein the hydrophilic polymer is hypromellose.

14. The formulation according to claim 1, wherein the dosage formulation comprises,
a) about 47 to 50 weight percent of a valproic acid compound;
b) about 13 to 16 weight percent hypromellose;
c) about 13 to 15 weight percent hydroxyethylcellulose;
d) about 8 to 9 weight percent pregelatinized starch;
e) about 4 to 10 weight percent microcrystalline cellulose;
f) about 3 to 4 weight percent silicon dioxide, and
g) about 1 to 2 weight percent stearic acid.

15. The formulation according to any preceding claim, having a dissolution profile in an aqueous buffer at 37°C and pH 5.5 of
a) no more than about 30% of total valproic acid compound is released during or within 3 hours;
b) from about 40 to about 70% of total valproic acid compound is released during or within 9 hours;
c) from about 50% to about 95% of total valproic acid compound is released during or within 12 hours, and;
d) not less than 75% of the total valproic acid compound is released during or within 18 hours.

16. The formulation according to any preceding claim, having a comparative pharmacokinetic profile compared to the pharmacokinetic profile of commercially available Divalproex Sodium ER 500mg tablets (Depakote ER) when dosed in a mammal of,
a) a mean AUC value in the range from about 90% to about 130%, wherein the observed mean AUC value for the Depakote ER is set at 100%, and
b) a mean Cₘₐₓ value in the range from about 100% to about 160% wherein the observed mean Cₘₐₓ value for the Depakote ER is set at 100%,
wherein the controlled release dosage formulation is administered to a fasting mammal.

17. The formulation according to any preceding claim, having a relative pharmacokinetic profile compared to the pharmacokinetic profile of commercially available Divalproex Sodium ER 500mg tablets (Depakote ER) in a mammal of,
a) a mean AUC value in the range from about 85% to about 120% wherein the observed mean AUC value for the Depakote ER is set at 100%,
b) a mean Cₘₐₓ value in the range from about 90% to about 160% wherein the observed mean Cₘₐₓ value for the Depakote ER is set at 100%,
wherein the controlled release dosage formulation is administered to a non-fasting mammal.

18. The formulation according to claims 16 or claim 17, wherein the mammal is a human.

19. The formulation of any preceding claim wherein the valproic acid compound is divalproex sodium.

20. A method of preparing a granular composition suitable for pressing into tablets of claim 1 comprising the following steps of,
a) dry blending a mixture comprising a valproic acid compound, at least two hydrophilic polymers, and binder;
b) wet granulating with a hydro alcoholic solution, a mixture of an alcohol and water, in order to form a homogeneous mixture;
c) drying and sizing the wet granulate;
d) surface coating the dried granulate with a dispersion containing a hydrophilic polymer and talc, with purified water;
e) drying the coated granulate;
f) optionally adding a glidant, and sieving the coated granulate;
g) optionally dry blending the coated granulate with a filler and a lubricant;
h) compressing the granules into tablets; and
i) optionally coating the tablets with a cosmetic coat wherein each hydrophilic polymer in steps a) and d) is in an amount of from 10 to 16% of the tablet weight.

21. The method according to claim 20, wherein the valproic acid compound is divalproex sodium, the at least two hydrophilic polymers are hypromellose and hydroxyethylcellulose, the binder is pregelatinized starch, the hydrophilic polymer for surface coating is hypromellose.

22. The method according to claim 21, wherein the valproic acid compound is divalproex sodium, the at least two hydrophilic polymers are hypromellose and hydroxyethylcellulose, the binder is pregelatinized starch, the hydrophilic polymer for surface coating is hypromellose, the glidant is silicon dioxide, the filler is microcrystalline cellulose, and the lubricant is stearic acid.

## Patentansprüche

1. Tablettenformulierung für kontrollierte Freisetzung, welche folgendes umfaßt:
a) eine Valproinsäureverbindung in einer Menge von etwa 40% bis etwa 80%, bezogen auf das Gewicht der Dosierungsform, und
b) wenigstens zwei hydrophile Polymere, jeweils in einer Menge von 10% bis 16% des Tablettengewichts,
wobei die Valproinsäureverbindung unter Valproinsäure oder einem pharmazeutisch verträglichen Salz, Ester oder Amid davon ausgewählt ist und die hydrophilen Polymere aus der Gruppe ausgewählt sind, bestehend aus Hypromellose (HPMC), Hydroxyethylzellulose (HEC), Polyethylenoxid, Polyvinylpyrrolidin, Hydroxypropylzellulose, Methylzellulose, Vinylacetat-Copolymeren, Polysacchariden, Methacrylsäure-Copolymeren und Maleinsäureanhydrid/Methylvinylether-Copolymeren.

2. Formulierung nach Anspruch 1, wobei die Valproinsäureverbindung in einer Menge von etwa 45% bis etwa 60% vorliegt.

3. Formulierung nach Anspruch 2, wobei die Valproinsäureverbindung in einer Menge von etwa 45% bis etwa 55% vorliegt.

4. Formulierung nach einem der vorangegangenen Ansprüche, wobei die Menge jedes hydrophilen Polymers von etwa 12% bis etwa 16% des Tablettengewichts beträgt.

5. Formulierung nach einem der vorangegangenen Ansprüche, wobei die hydrophilen Polymere aus der Gruppe ausgewählt sind, bestehend aus Hypromellose (Hydroxypropylmethylzellulose, HPMC), Hydroxyethylzellulose und Polyethylenoxid.

6. Formulierung nach einem der vorangegangenen Ansprüche, wobei die hydrophilen Polymere aus der Gruppe ausgewählt sind, bestehend aus Hypromellose (HPMC) und Hydroxyethylzellulose.

7. Formulierung nach einem der vorangegangenen Ansprüche, wobei die hydrophilen Polymere aus der Gruppe ausgewählt sind, bestehend aus Hypromellose K100 (Methocel K100), Hypromellose E15 (Methocel E15) und Hydroxyethylzellulose 250 (Natrosol^{®} 250M).

8. Formylierung nach einem der vorangegangenen Ansprüche, welche weiterhin wenigstens ein zusätzliches Polymer umfaßt, welches hydrophob ist.

9. Formulierung nach Anspruch 8, wobei das wenigstens eine hydrophobe Polymer Ethylzellulose ist.

10. Formulierung nach einem der vorangegangenen Ansprüche, wobei die Formulierung die Form eines beschichteten Kerns hat, welcher folgendes umfaßt:
a) ein komprimiertes oberflächenbeschichtetes Matrixgranulat und
b) eine Beschichtung auf dem komprimierten Kern.

11. Formulierung nach Anspruch 10, wobei das oberflächenbeschichtete Matrixgranulat die Valproinsäureverbindung und wenigstens zwei hydrophile Polymere umfaßt.

12. Formulierung nach einem der Ansprüche 10 bis 11, wobei die Oberflächenbeschichtung auf dem oberflächenbeschichteten Matrixgranulat ein hydrophiles Polymer umfaßt.

13. Formulierung nach Anspruch 12, wobei das hydrophile Polymer Hypromellose ist.

14. Formulierung nach Anspruch 1, wobei die Dosisformulierung folgendes umfaßt:
a) etwa 47 bis 50 Gewichtsprozent einer Valproinsäureverbindung,
b) etwa 13 bis 16 Gewichtsprozent Hypromellose,
c) etwa 13 bis 15 Gewichtsprozent Hydroxyethylzellulose,
d) etwa 8 bis 9 Gewichtsprozent vorgelatinierte Stärke,
e) etwa 4 bis 10 Gewichtsprozent mikrokristalline Zellulose,
f) etwa 3 bis 4 Gewichtsprozent Siliciumdioxid und
g) etwa 1 bis 2 Gewichtsprozent Stearinsäure.

15. Formulierung nach einem der vorangegangenen Ansprüche, mit einem Auflösungsprofil in einem wäßrigen Puffer bei 37°C und einem pH-Wert von 5,5, wobei
a) nicht mehr als etwa 30% der gesamten Valproinsäureverbindung während oder innerhalb von 3 Stunden freigesetzt werden,
b) von etwa 40 bis etwa 70% der gesamten Valproinsäureverbindung während oder innerhalb von 9 Stunden freigesetzt werden,
c) von etwa 50% bis etwa 95% der gesamten Valproinsäureverbindung während oder innerhalb von 12 Stunden freigesetzt werden und
d) nicht weniger als 75% der gesamten Valproinsäureverbindung während oder innerhalb von 18 Stunden freigesetzt werden.

16. Formulierung nach einem der vorangegangenen Ansprüche, mit einem vergleichbaren pharmakokinetischen Profil im Vergleich zu dem pharmakokinetischen Profil von kommerziell erhältlichen Divalproex Natrium ER 500 mg-Tabletten (Depakote ER) bei Dosierung in einem Säuger mit
a) einem mittleren AUC-Wert im Bereich von etwa 90% bis etwa 130%, wobei der beobachtete mittlere AUC-Wert für Depakote ER auf 100% festgesetzt wird, und
b) einem mittleren Cₘₐₓ-Wert im Bereich von etwa 100% bis etwa 160%, wobei der beobachtete mittlere Cₘₐₓ-Wert für Depakote ER auf 100% festgesetzt wird,
wobei die Dosisformulierung mit kontrollierter Freisetzung an einen fastenden Säuger verabreicht wird.

17. Formulierung nach einem der vorangegangenen Ansprüche, mit einem relativen pharmakokinetischen Profil im Vergleich zu dem pharmakokinetischen Profil von kommerziell erhältlichen Divalproex Natrium ER 500 mg-Tabletten (Depakote ER) in einem Säuger mit
a) einem mittleren AUC-Wert im Bereich von etwa 85% bis etwa 120%, wobei der beobachtete mittlere AUC-Wert für Depakote ER auf 100% festgesetzt wird,
b) einem mittleren Cₘₐₓ-Wert im Bereich von etwa 90% bis etwa 160%, wobei der beobachtete mittlere Cₘₐₓ-Wert für Depakote ER auf 100% festgesetzt wird,
wobei die Dosisformulierung mit kontrollierter Freisetzung an einen nicht-fastenden Säuger verabreicht wird.

18. Formulierung nach Anspruch 16 oder Anspruch 17, wobei der Säuger ein Mensch ist

19. Formulierung nach einem der vorangegangenen Ansprüche, wobei die Valproinsäureverbindung Divalproex-Natrium ist.

20. Verfahren zum Herstellen einer granulären Zusammensetzung, die zum Pressen zu Tabletten nach Anspruch 1 geeignet ist, welches die folgenden Stufen umfaßt
a) Trockenmischen eines Gemischs, welches eine Valproinsäureverbindung, wenigstens zwei hydrophile Polymere und Binder umfaßt,
b) Naßgranulieren mit einer alkoholisch-wäßrigen Lösung, einem Gemisch aus einem Alkohol und Wasser, unter Bildung eines homogenen Gemischs,
c) Trocknen und Größensortierung des nassen Granulats,
d) Beschichten der Oberfläche des getrockneten Granulats mit einer Dispersion, die ein hydrophiles Polymer und Talk enthält, mit gereinigtem Wasser,
e) Trocknen des beschichteten Granulats,
f) optional Zugeben eines Gleitmittels und Sieben des beschichteten Granulats,
g) optional Trockenmischen des beschichteten Granulats mit einem Füllmittel und einem Schmiermittel,
h) Komprimieren der Granalien zu Tabletten und
i) optional Beschichten der Tabletten mit einer kosmetischen Beschichtung,
wobei jedes hydrophile Polymer in den Stufen a) und d) in einer Menge von 10 bis 16% des Tablettengewichts vorliegt.

21. Verfahren nach Anspruch 20, wobei die Valproinsäureverbindung Divalproex-Natrium ist, die wenigstens zwei hydrophilen Polymere Hypromellose und Hydroxyethylzellulose sind, der Binder vorgelatinierte Stärke ist und das hydrophile Polymer für die Oberflächen beschichtung Hypromellose ist.

22. Verfahren nach Anspruch 21, wobei die Valproinsäureverbindung Divalproex-Natrium ist, die wenigstens zwei hydrophilen Polymere Hypromellose und Hydroxyethylzellulose sind, der Binder vorgelatinierte Stärke ist, das hydrophile Polymer für die Oberflächenbeschichtung Hypromellose ist, das Gleitmittel Siliciumdioxid ist, das Füllmittel mikrokristalline Zellulose ist und das Schmiermittel Stearinsäure ist.

## Revendications

1. Une formulation de comprimé à libération controlée comprenant,
a) un composé à base d'acide valproïque en une quantité d'environ 40% à environ 80% en poids de la forme pharmaceutique, et
b) au moins deux polymères hydrophiles employés chacun en une quantité de 10% à 16% du poids du comprimé,
dans laquelle le composé à base d'acide valproïque est choisi dans le groupe comprenant l'acide valproïque, ou un sel, ester ou amide pharmaceutiquemment acceptable en dérivant, et les polymères hydrophiles sont choisis dans le groupe comprenant l'hypromellose (HPMC), l'hydroxyethyl cellulose (HEC), l'oxyde de polyéthylène, la polyvinylpyrrolidine, l'hydroxypropyl cellulose, la méthyl cellulose, les copolymères d'acétate de vinyle, les polysaccharides, les copolymères d'acide méthacrylique et les copolymères d'anhydride maleique et de méthyl vinyl ether.

2. La formulation selon la revendication 1, dans laquelle le composé à base d'acide valproïque est présent en une quantité d'environ 45% à environ 60%

3. La formulation selon la revendication 2, dans laquelle le composé à base d'acide valproïque est présent en une quantité d'environ 45% à environ 55%.

4. La formulation selon une quelconque des revendications précédentes, dans laquelle la quantité de chacun des polymères hydrophiles est comprise entre environ 12% et environ 16% du poids du comprimé

5. La formulation selon une quelconque des revendications précédentes, dans laquelle les polymères hydrophiles sont choisis dans le groupe comprenant l'hypromellose (hydroxypropylmethyl cellulose, HPMC), l'hydroxyethyl cellulose et l'oxyde de polyéthylène.

6. La formulation selon une quelconque des revendications précédentes, dans laquelle the polymères hydrophiles sont choisis dans le groupe comprenant l'hypromellose (HPMC) et l'hydroxyéthylcellulose.

7. La formulation selon une quelconque des revendications précédentes, dans laquelle les polymères hydrophiles sont choisis dans le groupe comprenant l'hypromellose K100 (Methocel K100), l'hypromellose E15 (Methocel E15), et l'hydroxyethylcellulose 250 (Natrosol® 250M).

8. La formulation selon une quelconque des revendications précédentes, comprenant en outre au moins un polymère supplémentaire ayant des propriétés hydrophobes.

9. La formulation selon la revendication 8, dans laquelle au moins un polymère hydrophobe est l'éthylcellulose.

10. La formulation selon une quelconque des revendications précédentes,
dans laquelle la formulation est sous forme d'un noyau revêtu, comprenant
a) une matrice, formée d'un granulat compressé, dont la surface est revêtue, et
b) un revêtement sur le noyau compressé.

11. La formulation selon la revendication 10, dans laquelle la matrice, formée d'un granulat compressé, dont la surface est revêtue, comprend le composé à base d'acide valproïque et au moins deux polymères hydrophiles.

12. La formulation selon une quelconque des revendications 10 ou 11, dans laquelle le revêtement de surface de la matrice, formée d'un granulat compressé, dont la surface est revêtue, comprend un polymère hydrophile.

13. La formulation selon la revendication 12, dans laquelle le polymère hydrophile est l'hypromellose.

14. La formulation selon la revendication 1, dans laquelle la formulation pharmaceutique comprend,
a) environ 47 à 50% en poids d'un composé à base d'acide valproïque;
b) environ 13 à 16% en poids d'hypromellose;
c) environ 13 à 15% en poids d'hydroxyethylcellulose;
d) environ 8 à 9% en poids d'amidon prégélatinisé;
e) environ 4 à 10% en poids de cellulose microcrystalline;
f) environ 3 à 4% en poids de dioxyde de silice, et
g) environ 1 à 2% en poids d'acide stéarique.

15. La formulation selon une quelconque des revendications précédentes, présentant un profil de dissolution, dans un tampon aqueux à 37°C et à un pH de 5,5, tel que
a) pas plus d'environ 30% de l'ensemble du composé à base d'acide valproïque est libéré durant ou en 3 heures;
b) entre environ 40 à environ 70% de l'ensemble du composé à base d'acide est libéré durant ou en 9 heures;
c) entre environ 50% à environ 95% de l'ensemble du composé à base d'acide est libéré durant ou en 12 heures, et;
d) pas moins de 75% de l'ensemble du composé à base d'acide valproïque est libéré durant ou en 18 heures.

16. La formulation selon une quelconque des revendications précédentes, présentant un profil pharmacocinétique comparé par comparaison avec le profil pharmacocinétique des comprimés de 500 mg de Divalproex Sodium ER disponibles dans le commerce (Depakote ER), quant ils sont administrés à un mammifère, de:
a) une valeur AUC moyenne comprise entre environ 90% et environ 130%, lorsque la valeur AUC moyenne observée moyenne pour le Depakote ER est fixée à 100%, et
b) une valeur Cₘₐₓ moyenne comprise entre environ 100% et environ 160% lorsque la valeur Cₘₐₓ moyenne observée pour le Depakote ER est fixée à 100%.
dans laquelle la formulation pharmaceutique à liberation contrôlée est administrée à un mammifère qui jeûne.

17. La formulation selon l'une quelconque des revendications précédentes, présentant un profil ayant une pharmacocinétique relative comparée au profil pharmacocinétique de comprimés de 500 mg de Divalproex Sodium ER disponibles dans le commerce (Depakote ER) quant administré à un mammifère de:
a) une valeur AUC moyenne comprise entre environ 85% et environ 120%, lorsque la valeur AUC moyenne observée moyenne pour le Depakote ER est fixée à 100%, et
b) une valeur Cₘₐₓ moyenne comprise entre environ 90% et environ 160%lorsque la valeur Cₘₐₓ moyenne observée pour le Depakote ER est fixée à 100%.
dans laquelle la formulation pharmaceutique à liberation contrôlée est administrée à un mammifère qui ne jeûne pas.

18. La formulation selon la revendications 16 ou la revendication 17, dans laquelle le mammifère est un homme.

19. La formulation selon une quelconque des revendications précédentes dans laquelle le composé à base d'acide valproïque est le divalproex sodium.

20. Un procédé de préparation d'une composition granulaire, susceptible par compression d'être mises sous forme de comprimés selon la revendication 1, comprenant les étapes suivantes de:
a) mélange à sec d'un mélange comprenant un composé à base d'acide valproïque, au moins deux polymères hydrophiles, et un liant;
b) granulation par voie humide dans un solution hydro-alcoolique d'un mélange d'alcool et d'eau, afin de former un mélange homogène;
c) séchage et calibrage des granules humides;
d) revêtement de la surface des granules séchés dans une dispersion contenant un polymère hydrophile et du talc, avec de l'eau purifiée;
e) séchage des granules revêtus;
f) éventuellement addition d'un glissant et tamisage des granules revêtus;
g) éventuellement mélange à sec des granules revêtus avec une charge et un lubrifiant;
h) compression des granules en comprimés;
i) éventuellement revêtement des comprimés par un revêtement cosmétique.
dans lequel chacun des polymères hydrophiles des étapes a) et b) est présent en une quantité comprise entre 10 et 16% du poids des comprimés.

21. Le procédé selon la revendication 20, dans lequel le composé à base d'acide valproïque est le divalproex sodium, les au moins deux polymères hydrophiles sont l'hypromellose et l'hydroxyethylcellulose, le liant est de l'amidon prégélatinisé, le polymère hydrophile pour le revêtement de surface est de l'hypromellose.

22. Le procédé selon la revendication 21, dans lequel le composé à base d'acide valproïque est le divalproex sodium, les au moins deux polymères hydrophiles sont l'hypromellose et l'hydroxyethylcellulose, le liant est de l'amidon prégélatinisé, le polymère hydrophile pour le revêtement de surface est de l'hypromellose, le glidant est du dioxyde de silicium, la charge est de la cellulose microcrystalline et le lubrifiant est de l'acide stearique.
